# EUROPEAN PATENT SPECIFICATION

(11) **EP 2 461 827 B1**
(45) Date of publication and mention of the grant of the patent: **10.08.2016**
(21) Application number: 10737125.4
(22) Date of filing: 24.06.2010
(51) Int. Cl.: A61K 39/385, A61K 39/145, C07K 14/075, C07K 14/395

(54) **VIRUS-LIKE PARTICLE VECTOR AS A POLYVALENT PLATFORM FOR INTRACELLULAR DELIVERY OF HIGH-MOLECULAR-WEIGHT THERAPEUTIC SUBSTANCES, METHOD FOR GENERATING A VIRUS LIKE PARTICLE VECTOR AND USE OF A VIRUS-LIKE PARTICLE VECTOR AND A PHARMACEUTICAL COMPOSITION CONTAINING SAID VIRUS-LIKE PARTICLE VECTOR**
VLP-VEKTOR ALS POLYVALENTE PLATTFORM FÜR DIE INTRAZELLULÄRE FREISETZUNG VON THERAPEUTISCHEN STOFFEN MIT HOHEM MOLEKULARGEWICHT, VERFAHREN ZUR HERSTELLUNG EINES VLP-VEKTORS UND VERWENDUNG EINES VLP-VEKTORS SOWIE EINE PHARMAZEUTISCHE ZUSAMMENSETZUNG MIT DIESEM VLP-VEKTOR
VECTEUR DE PARTICULES DE TYPE VIRUS COMME PLATEFORME POLYVALENTE POUR UNE DISTRIBUTION INTRACELLULAIRE DE SUBSTANCES THÉRAPEUTIQUES DE POIDS MOLÉCULAIRE ÉLEVÉ, PROCÉDÉ POUR GÉNÉRER UN VECTEUR DE PARTICULES DE TYPE VIRUS ET UTILISATION D'UN VECTEUR DE PARTICULES DE TYPE VIRUS ET COMPOSITION PHARMACEUTIQUE CONTENANT LEDIT VECTEUR DE PARTICULES DE TYPE VIRUS

(30) Priority: 26.06.2009 PL 38840309
(43) Date of publication of application: 13.06.2012
(73) Proprietor: Instytut Biochemii I Biofizyki, 02-106 Warszawa (PL)
(72) Inventor: SZOLAJSKA, Ewa, 00-189 Warszawa (PL); CHROBOCZEK, Jadwiga, 02-564 Warszawa (PL); NASKALSKA, Antonina, 31-139 Kraków (PL)
(74) Representative: Twardowska, Aleksandra
(86) International application number: PCT/PL2010/000051
(87) International publication number: WO 2010/151159

(56) References cited:
- WO-A2-02/00885
- GARCEL A ET AL: "Protein transduction into human cells by adenovirus dodecahedron using WW domains as universal adaptors" JOURNAL OF GENE MEDICINE, vol. 8, no. 4, April 2006 (2006-04), pages 524-531, XP002607201
- SHCHERBIK NATALIA ET AL: "Substrate proteolysis is inhibited by dominant-negative Nedd4 and Rsp5 mutants harboring alterations in WW domain 1" JOURNAL OF CELL SCIENCE, vol. 115, no. 5, 1 March 2002 (2002-03-01), pages 1041-1048, XP002607202 ISSN: 0021-9533
- HARTY RONALD N ET AL: "Rhabdoviruses and the cellular ubiquitin-proteasome system: A budding interaction" JOURNAL OF VIROLOGY, vol. 75, no. 22, November 2001 (2001-11), pages 10623-10629, XP002607203 ISSN: 0022-538X
- FENDER P ET AL: "Adenovirus dodecahedron allows large multimeric protein transduction in human cells." JOURNAL OF VIROLOGY, vol. 77, no. 8, April 2003 (2003-04), pages 4960-4964, XP002607204 ISSN: 0022-538X cited in the application
- NASKALSKA A ET AL: "Influenza recombinant vaccine: Matrix protein M1 on the platform of the adenovirus dodecahedron" VACCINE, vol. 27, no. 52, December 2009 (2009-12), pages 7385-7393, XP26789498 ISSN: 0264-410X DOI: DOI:10.1016/J.VACCINE.2009.09.021
- SZURGOT INGA ET AL: "Self-adjuvanting influenza candidate vaccine presenting epitopes for cell-mediated immunity on a proteinaceous multivalent nanoplatform", VACCINE, vol. 31, no. 40 , pages 4338-4346, XP028697958, ISSN: 0264-410X, DOI: 10.1016/J.VACCINE.2013.07.021

### SEQUENCE LISTING

<110> Instytut Biochemii i Biofizyki PAN Chroboczek , Jadwiga Naskalska, Antonina Szo 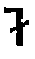 ajska, Ewa
<120> virus-like particle vector as a polyvalent platform for intracellular delivery of high-molecular-weight therapeutic substances, method for generating a virus like particle vector, use of a virus like particle vector, pharmaceutical composition
<130> 9461/09/AT
<140> PCT/PL2010/
   <141> 2010-06-18
<150> PL20090388403
   <151> 2009-06-26
<160> 18
<170> PatentIn version 3.3
<210> 1
   <211> 26
   <212> DNA
   <213> Artificial
<220>
   <223> WW1,2,3 derived from yeast Rsp5 protein
<400> 1
   ttattcgaac cgccctgggg tcctga 26
<210> 2
   <211> 31
   <212> DNA
   <213> Artificial
<220>
   <223> ww1,2,3 derived from yeast Rsp5 protein
<400> 2
   atgatggcgc taggtgttcg agcttacgta g 31
<210> 3
   <211> 31
   <212> DNA
   <213> Artificial
<220>
   <223> the gene coding for matrix protein 1 (M1) from influenza (Puerto Rico/8/34 H1N1)
<400> 3
   ggacggtcga aactcttctg agtatccaat t 31
<210> 4
   <211> 31
   <212> DNA
   <213> Artificial
<220>
   <223> gene coding for matrix protein 1 (M1) from influenza (Puerto Rico/8/34 H1N1), originally provided *in* pET15b
<400> 4
   ggacggtcga aactcttctg agtatccaat t 31
<210> 5
   <211> 29
   <212> DNA
   <213> Artificial
<220>
   <223> WW1,2,3 derived from yeast Rsp5 protein, originally placed in pGEX-4T-1 plasmid
<400> 5
   cttcgctagc attcttaccc cctggttgg 29
<210> 6
   <211> 37
   <212> DNA
   <213> Artificial
<220>
   <223> WW1,2,3 derived from yeast Rsp5 protein, originally placed in pGEX-4T-1 plasmid
<400> 6
   cacgagcatg ccgctacata tggtctagcg atgatgg 37
<210> 7
   <211> 24
   <212> DNA
   <213> Artificial
<220>
   <223> the gene coding for hemagglutinin HAO of avian influenza A virus (A/swan/Poland/467/2006/H5N1) and ww1,2,3 derived from yeast Rsp5
<400> 7
   cagggcatat ggagaaaata gtgc 24
<210> 8
   <211> 23
   <212> DNA
   <213> Artificial
<220>
   <223> The gene coding for hemagglutinin HAO of avian influenza A virus (A/swan/Poland/467/2006/H5N1) and WW1,2,3 derived from yeast Rsp5
<400> 8
   aactcgcatg cttaaatgca aat 23
<210> 9
   <211> 30
   <212> DNA
   <213> Artificial
<220>
   <223> the gene coding for hemagglutinin HAO of avian influenza A virus (A/swan/Poland/467/2006/H5N1) and WW1,2,3 derived from yeast Rsp5
<400> 9
   ggtaagctcg agaaatggag aaaatagtgc 30
<210> 10
   <211> 29
   <212> DNA
   <213> Artificial
<220>
   <223> the gene coding for hemagglutinin HAO of avian influenza A virus (A/swan/Poland/467/2006/H5N1) and ww1,2,3 derived from yeast Rsp5
<400> 10
   gaacgctagc atagaaatgc aaattctgc 29
<210> 11
   <211> 41
   <212> DNA
   <213> Artificial
<220>
   <223> the gene coding for hemagglutinin HAO of avian influenza A virus (A/swan/Poland/467/2006/H5N1) and WW1,2,3 derived from yeast Rsp5
<400> 11
   ccatcatcgc tagaccatat gagtgatcag atttgcattg g 41
<210> 12
   <211> 30
   <212> DNA
   <213> Artificial
<220>
   <223> the gene coding for hemagglutinin HA0 of avian influenza A virus (A/swan/Poland/467/2006/H5N1) and WW1,2,3 derived from yeast Rsp5
<400> 12
   gcgttgcatg ctattggtag gttcctattg 30
<210> 13
   <211> 28
   <212> DNA
   <213> Artificial
<220>
   <223> the gene coding for hemagglutinin HA0 of avian influenza A virus (A/swan/Poland/467/2006/H5N1) and WW1,2,3 derived from yeast Rsp5
<400> 13
   cagcctcgag atgagtgatc agatttgc 28
<210> 14
   <211> 27
   <212> DNA
   <213> Artificial
<220>
   <223> the gene coding for hemagglutinin HA0 of avian influenza A virus (A/swan/Poland/467/2006/H5N1) and WW1,2,3 derived from yeast Rsp5
<400> 14
   caggtgctag ctggtaggtt cctattg 27
<210> 15
   <211> 30
   <212> DNA
   <213> Artificial
<220>
   <223> the gene coding for hemagglutinin HA0 of avian influenza A virus (A/swan/Poland/467/2006/H5N1) and ww1,2,3 derived from yeast Rsp5
<400> 15
   ggtaagctcg agaaatggag aaaatagtgc 30
<210> 16
   <211> 27
   <212> DNA
   <213> Artificial
<220>
   <223> the gene coding for hemagglutinin HA0 of avian influenza A virus (A/swan/Poland/467/2006/H5N1) and ww1,2,3 derived from yeast Rsp5
<400> 16
   caggtgctag ctggtaggtt cctattg 27
<210> 17
   <211> 41
   <212> DNA
   <213> Artificial
<220>
   <223> the gene coding for hemagglutinin HA0 of avian influenza A virus (A/swan/Poland/467/2006/H5N1) and ww1,2,3 derived from yeast Rsp5
<400> 17
   ccatcatcgc tagaccatat gagtgatcag atttgcattg g 41
<210> 18
   <211> 30
   <212> DNA
   <213> Artificial
<220>
   <223> the gene coding for hemagglutinin HA0 of avian influenza A virus (A/swan/Poland/467/2006/H5N1) and WW1,2,3 derived from yeast Rsp5
<400> 18
   gcgttgcatg ctattggtag gttcctattg 30

## Description

The disclosure relates to a virus-like particle (VLP) vector, a method for production of said vector, application of said vector and a pharmaceutical composition containing said virus-like particle vector. More particularly, the disclosure relates to a protein virus-like particle vector composed of an adenovirus dodecahedron (Dd) and delivering therapeutic substances belonging to classes of proteins, polysaccharides, nucleic acids, lipids, lipoproteins or derivatives thereof, to mammalian cells. This disclosure allows for production of human or animal vaccines, delivery of antibodies, antitumour proteins and immunosuppressive agents, as well as proteins/peptides and lipids determining specific tissue tropism or nucleic acids. The method of the disclosure involves the therapeutic substances being inserted in polyvalent manner to different sites within the Dd, such as N- or C-terminus or various external loops or by means of interactions with the binding site of the Dd viral partner - adenovirus fibre protein, interactions with the RGD (arginine-glycine-aspartic acid) motif or with the basic or hydrophobic fragments of the vector, non-covalently attached by means of ionic bonds or hydrophobic interactions, or covalently tethered, e.g. by means of cross-linking reactions. The invention involves the therapeutic substances comprising an antigen and attached through interactions of vector PPxY motifs with linker containing WW domains, where the WW domains are derived from fungal proteins, especially from the proteins of yeast strains.

Adenovirus (Ad) is a nonenveloped human and animal virus consisting of a single double-strand DNA molecule and 11-14 virus-coded proteins. The capsid of the adenovirus is a icosahedron with 20 faces built of hexons and 12 vertices built of pentons. The penton is a non-covalent complex of two proteins - a pentameric penton base protein and a trimeric fibre protein, as shown below.

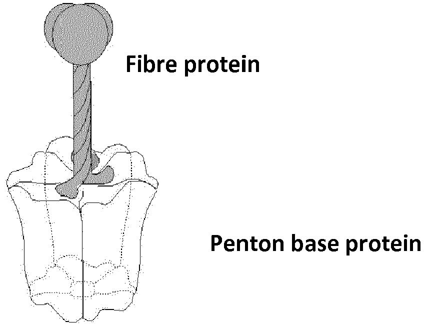

The pentons play a dual role in initiation of viral infection. The infection starts with the adsorption of the virus, where the infecting virion binds the primary receptor at the surface of susceptible cells using the C-terminal globular fibre domain (Philipson et al., 1968). The second stage of viral infection is virus penetration at which is involed the penton base protein. Virus penetration results from interaction between the penton base RGD motif and the cellular integrins (Wickham et al, 1993.) The penton base protein is also implicated in the release of the virus from the endosomes. Acidic environment within the endosomes is believed to result in a structural modification resulting in the emergence of hydrophobic domains of the penton base protein followed by interaction between these domains and the endosomal membrane, which leads to the membrane being broken (Wohlfart, 1988). The tissue specificity (or tropism) of adenoviruses depends on the fibre protein. Thus, for example, adenoviruses of subgroup B (Ad3, Ad7) most commonly infect the respiratory tract and the eyes, adenoviruses of subgroup C (Ad2, Ad5) infect the respiratory tract, while adenoviruses of subgroup F (Ad40, Ad41) infect the digestive tract.

Upon normal infection, some adenovirus serotypes generate symmetrical, dodecahedral virus-like particles devoid of the viral genome and formed of 12 copies of pentons responsible for virus penetration. Except for electron microscopic images published long time ago (Valentine and Pereira, 1965), nothing is known about the function, role or molecular biology of these virus-like particles. Overproduction of penton base protein and fibre protein derived from adenovirus of serotype 3 in baculovirus system results in spontaneous formation of identical symmetrical dodecahedral particles comprised of 12 pentons. Expression of the penton base protein in the baculovirus system results in formation of dodecahedral virus-like particles formed of 12 copies of penton base protein only. Both types of dodecahedra maintain the functionality of its components, showing an exceptional cell penetration capability (Fender et al., 1997; Fender et al., 2003; Vives et al., 2004).

The dodecahedron can recognize two types of receptors. On one hand, it retains the specificity of the penton base protein recognizing αv integrins, which are highly expressed on neoplastic vessels that deliver blood to the tumour tissue. At the same time, the dodecahedron has strong affinity towards heparan sulphates (Vives et al., 2004), which are located at the surface of all epithelial cells.

Patents no. FR 2,747,681 (published 1997/10/24) and FR 2,741,087 (published 1997-05-16) disclose a protein adenovirus dodecahedron complex consisting of penton base protein and optionally containing a fibre protein. Adenoviral protein complex (A) comprises: (a) 12 pentons (P), each containing at least one each of penton base (Pb) and fibre protein (F), with pentons bound via the Pb to form a dodecahedral structure that is resistant to proteolysis and has molecular weight of 4.8-6.6 MD; or (b) 12 Pb forming a dodecahedron as described above but with molecular weight of 3.2-4 MD. (A) contains no other components of the adenoviral genome and F and Pb are derived from the same or different adenovirus (Ad) serotypes.

Patents no. US 6,083,720 (published 2000/07/04), EP 0 861 329 (published 2000/07/04), WO 9 718 317 (published 1997/05/22) disclose an adenovirus dodecahedron protein complex, a composition containing said protein complex, and an application thereof. A native or recombinant adenoviral protein complex can be used for treating and preventing human and animal diseases. The adenoviral protein complex consists of 12 pentons, each including at least one fibre protein and twelve penton base proteins without any other adenoviral elements, with said fibre protein being derived from one or more adenoviruses serotypes and said penton base proteins being bound together to form a proteolytic enzyme-stable dodecahedral structure.

Patent no. PL 192,016 (published 1999/11/30) discloses a composition and a method for generation of an artificial, ordered and repeatable antigen system, a vaccine composition and an application thereof. The document provides a method for generating ordered and repeatable antigen systems or antigen determinants. The compositions are useful in vaccines for prevention of infectious diseases, in treatment of allergies and in cancer treatment. Different embodiments in the document provide a virus, a virus-like particle, a viral capsid particle, phage or recombinant forms thereof, coated in an orderly and repeatable fashion by an antigen as a result of specific interactions. In a particular embodiment in the document the new technology allows for generation of antigen-coated virus particles. Other particular embodiments allow for generation of antigen-coated Hepatitis B virus-like particles or antigen-coated measles virus-like particles.

Patent no PL366990 (published 2005/02/07) discloses a novel vaccine composition.

The described embodiment relates to an inactivated influenza virus preparation containing an external viral antigen - hemagglutinin - stabilized without the use of thiomersalate or with low-dose thiomersalate. The influenza vaccine preparation may contain a micelle-modifying excipient, such as α-tocopherol or a derivative thereof, in an amount sufficient for hemagglutinin stabilization.

Patent no. WO 0,166,700 (published 2001/09/13) discloses a subcutaneous immunization method for large molecular antigens. WO0166700 discloses non-invasive, economical and easy methods for inducing an immune response against a particulate antigen.

Patents no. EP 1,862,550 (published 2007/12.05) and US 2,005,186,621 (published 2005/08/25) disclose formation of virus-like particles derived from wild type influenza virus and chimeric influenza virus-like particles. Influenza virus-like particles (VLPs) built of structural proteins: hemagglutinin (HA), neuraminidase (NA), and matrix proteins M1 and M2, are described. VLPs can also be generated from M1 alone, or from M1 in combination with HA, NA and M2.

Patent no. WO 2007/085/969 (published 2007/08/02) discloses influenza vaccines comprising hemagglutinin (HA) and matrix proteins (M1 and M2). The document provides an immunogenic anti-flu composition comprising HA and M1 and M2, being derived from viruses grown in cell culture rather than eggs. The matrix proteins may be a fragment of a full-length influenza virus matrix protein, namely a fragment of M1 matrix protein with a molecular weight of less than 20 kDa. The composition may be a subunit vaccine comprising purified viral surface glycoproteins.

Patent no. WO 2007/066/334 (published 2007/06/14) relates to influenza vaccines for human and veterinary use, in particular to a vaccine able to induce long term and cross-strain protection comprising at least two influenza virus epitopes expressed as a chimeric polypeptide, wherein at least one epitope is influenza A virus matrix protein epitope (M1 or M2), and the second epitope is a haemagglutinin peptide epitope. Patent no. CA 2,615,372 (published 2009/01/13) discloses influenza virus-like particles comprising hemagglutinin (HA) and a method for synthesis of these particles within a plant or a plant fragment. The said method involves expression of influenza virus HA in the plant and HA purification. The document relates also to VLPs comprising influenza virus HA, plant lipids and pharmaceutically acceptable carriers. The VLPs of the document may be used in the development of vaccines or for improvement of vaccines developed to date.

Patent no. WO 2009/012/489 (published 2009/01/22) discloses chimeric avian influenza VLPs. The document provides a method for increasing the yield of production of VLPs containing avian influenza matrix protein. The document also relates to methods of making and using said VLPs.

Despite the studies discussed above, there is a constant need to develop an efficient virus-like particle vector. To this end, the inventors have developed a polyvalent protein vector, an adenovirus dodecahedron (Dd) delivering therapeutic substances to mammalian cells. A recombinant dodecahedral particle rDd is generated in insect cells in a baculovirus system with a high yield of 10 mg rDd per 100 ml of cell culture. Such a yield is comparable to that achieved in the most efficient bacterial systems. To date, rDd has been purified only by ultracentrifugation in sucrose gradient. This stage allows for elimination of cellular proteins, but not of nucleic acids, which are most probably tethered to the Dd surface.

The goal of this invention was to develop conditions allowing for using a dodecahedral virus-like particle vector to transport, in a polyvalent manner, such therapeutic agents as proteins, polysaccharides, nucleic acids, lipids, lipoproteins or derivatives thereof, such as human or veterinary vaccines, antibodies, antitumour peptides, as well as immunosuppressive substances or proteins/peptides and lipids determining tissue tropism, into mammalian cells, including human cells. In their work, the Applicants found that the dodecahedron is more efficient in cell penetration than the adenovirus of serotype 3 (Ad3), from which the dodecahedron is derived.

This vector transduces 100% of cells in cell culture, being also able to transduce cells that are non-permissive with respect to Ad3. It follows that compared to its virus of origin, Dd shows gain-of-function, namely that of being capable of recognizing heparan sulphates. Heparan sulphates (HS) interact with positively-charged protein fragments; it seems that the neighbouring penton bases form sites responsible for interactions with HS. In viral capsids, the penton bases are more distant due to the presence of another viral protein, hexon, between the neighbouring penton bases.

Therefore, the dodecahedron penetration is not only mediated by virus receptors, but can also be aided by heparan sulphates, which are present at the surface of all epithelial cells.

Dodecahedron is potentially a vector, which may act as a polyvalent platform for antigen delivery. Due to its the remarkable level of intracellular penetration and to the polyvalence of the generated vaccine, the use of dodecahedron as a delivery platform would allow for induction of significant responses of the animal/human immune system, which could allow to prevent epidemics of infectious diseases, such as influenza.

The dodecahedron is also a potential tumour cell-specific vector. It shows an endosomolytic activity typical of adenoviruses, resulting in very efficient penetration and liberation into the cytosol. In addition, the vector has high affinity towards αv integrins, which recognize the RGD (arginine-glycine-aspartic acid) motif of penton bases. Adenovirus dodecahedron built of 60 penton base polypepeptide chains and thus comprising 60 RGD motifs, is perhaps the most specific αv integrin ligand. Since the levels of αv integrins is known to be elevated in the endothelium of the malignant tumours, it is conceivable that Dd could selectively deliver therapeutic agents into the endothelial cells of the newly formed tumour blood vessels. Therefore, the inventors decided to use said virus-like particles in transporting therapeutic agents such as anticancer proteins or peptides.

The invention in its broadest sense is as defined in the independent claims.

This invention achieves the above goal and provides solution to the problems described in current art and associated with the development of an invention allowing for an efficient introduction of a protein or peptide therapeutic substance comprising an antigen into the cell to achieve its functionality. Globular WW domains with the length of 23-30 amino acids are the smallest known protein domains. Their name derives from two conserved tryptophan residues (tryptophan is denoted as W in the one letter amino acid code) that are essential for appropriate domain folding and ligand binding (Koepf et al., 1999). The WW domains are capable of binding proline-rich peptides having specific consensus sequences (Pawson and Scott, 1977). One of such sequences is the PPxY (proline - proline - any amino acid - tyrosine) motif. Adenoviral base protein contains two such conserved motifs in the N-terminal part. The Applicants found that one of these motifs is responsible for interactions with WW domains (Galinier et al., 2002).

According to the invention, therapeutic substances being genetically fused to the WW domains, are non-covalently tethered to the vector in a polyvalent manner by means of interactions with the PPxY vector motifs.

This invention relates to a novel influenza vaccine built of adenovirus dodecahedron which forms a platform for the delivery of two influenza virus antigens as defined in the claims: the internal protein known as M1 matrix protein, and the external protein, hemagglutinin (HA). WW domains linker co-acting with the Dd was used to tether the antigen to the vector. Internalization of the complex of the Dd and the WW domain-fused antigens was demonstrated using biochemistry and molecular biology techniques. At the next stage, antigen M1 in complex with Dd was demonstrated to be a strong activator of human myeloid dendritic cells (MDCs) and to be efficiently presented by the dendritic cells to the M1-specific CD8+ T lymphocytes. Simultaneously, a HA variant with hemagglutination activity was obtained, suggesting a proper structure of the antigen. Similarly as above, HA in fusion with WW linker was prepared, attached to Dd and shown to be delivered to cells in vitro. These results suggest that the proposed vaccine model is fully feasible and that the adenovirus dodecahedron is an efficient vector for transporting foreign antigens into human cells and an effective platform for antigen presentation.

The subject matter of invention is a virus-like particle vector characterized in that said vector is a recombinant adenovirus dodecahedron (Dd) particle consisting of pentons or penton base proteins from a mammalian adenovirus, particularly from an human adenovirus, which Dd carries non-covalently attached fusion proteins comprising multiple WW domains from fungal proteins, especially from yeast proteins, preferably from *S. cerevisiae* strain protein, preferably from Rsp5 protein, genetically fused to a protein or peptide therapeutic substance comprising an antigens, preferably an influenza virus antigen, and wherein said WW domains interact with PPxY (proline-pro line-any amino acid-tyrosine) motifs of the Dd.

Preferably, three WW domains derived from Rsp5 protein act in tandem as universal adapters allowing multiple copies of any protein or peptide to be tethered to the dodecahedra.

Preferably, the fusion proteins have the WW domains at their C-terminus.

Preferably, the antigen protein is influenza virus matrix protein M1 or hemagglutinin HA, or fragments thereof, genetically fused to the WW linker protein.

The next subject of invention is a method for generating a virus like particle vector described above, characterized in that the recombinant adenovirus dodecahedron particle consisting of pentons or penton base proteins from a mammalian, particularly human, adenovirus, is generated in insect cells in a baculovirus system and subsequently purified using sucrose density gradient and ion exchange column to eliminate low- and medium-molecular proteins and nucleic acids and to obtain an rDd fraction with purity of more than 90%; next, multiple copies of fusion protein including WW domains from fungal protein, particularly from yeast protein, extended by a protein- or peptide-based therapeutic substance, preferably a human or animal vaccine, an anti-tumour, immunogenic or immunosuppressive peptide and optionally fusion protein including a WW domain from fungal protein, particularly from yeast protein, extended by a peptide/protein, lipid or polysaccharide determining specific tissue tropism, are attached by *in vitro* incubation or by simultaneous expression in the baculovirus system.

Preferably, the protein- or peptide therapeutic substance comprises an M1 or HA influenza virus antigen, or fragments thereof.

Preferably, said fusion protein consists of influenza proteins M1, HA, or fragments thereof, genetically fused to a WW linker protein.

Preferably, the recombinant dodecahedron particle, rDd, is obtained in high yield in insect cells in a baculovirus system, wherein the yield is at least 8 mg rDd per 100 ml of cell culture.

The next subject of invention is a virus-like particle vector according to the above for use in the delivery of therapeutic substances to human or animal tissues. Preferably, the protein or peptide therapeutic substance comprises an M1 or HA influenza virus antigen, or fragments thereof.

The next subject of invention is a pharmaceutical composition, characterized in that said composition contains a virus-like particle vector described above. Preferably, said composition is in the form of vaccine.

The enclosed figures allow to better explain the nature of the invention, where:

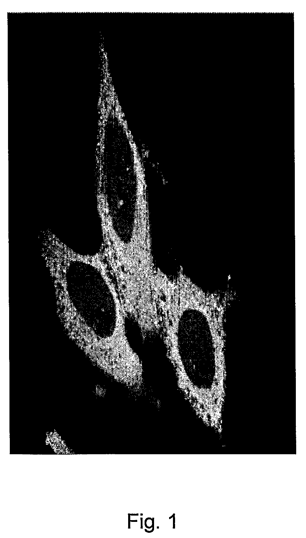

**Figure 1** presents the penetration of human HeLa cells by the dodecahedra in an *in vitro* culture. Cells treated with Dds were analysed under confocal microscope with anti-Dd antibody - green cytoplasmic signal (white/grey in black-and-white images). Cell nuclei were stained blue using DAPI (marked grey in black-and-white images).

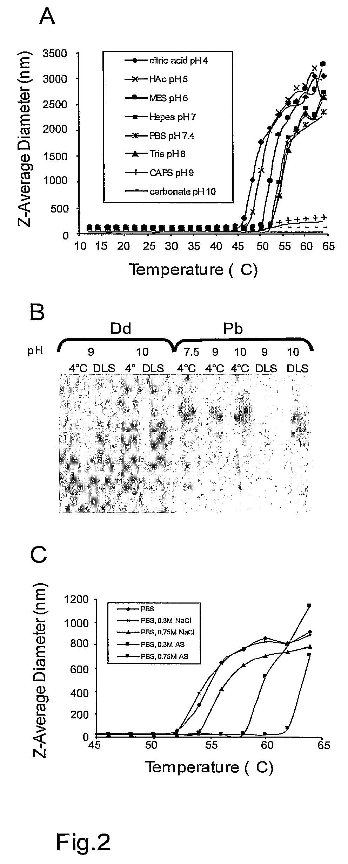

**Figure 2** presents thermal stability of Dds as a function of pH and ionic strength. Dds were analysed by dynamic light scattering (DLS) at different pH in the presence of 150 mM NaCl, at temperature increments of 2°C measured every 2 min. in the range of 12 to 65°C. **(B)** Analysis of Dd and pentameric bases (Pb) in CAPS buffer at pH 9 and in carbonate buffer at pH 10. Some samples were subjected to temperature treatment imitating DLS conditions. **(C)** DLS analysis carried out on Dds in PBS under different ionic strength conditions. Mean values of three apparatus readings are presented.

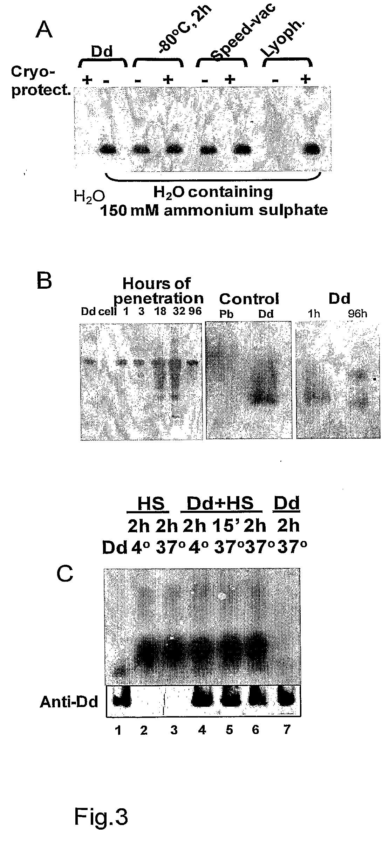

**Figure 3** presents the stability of Dds upon lyophilization in HeLa cells and in human serum. **(A)** Purified rDds were dialysed overnight at 4°C against water or 150 mM aqueous ammonium sulphate solution. Mannitol (0,4%) and sucrose (0.4%) were added to samples marked "Cryoprotect +". Dd samples were frozen at -80°C, dried in speed-vac, or lyophilized. Dry samples were reconstituted in the starting volume of water. Samples were centrifuged for 30 min. at 13,000 rpm, and the oligomeric status of proteins in the supernatant was determined by agarose gel electrophoresis. **(B)** Stability of Dds in HeLa cell culture. Purified rDds (2 µg/100 µl) were applied onto 2x10⁴ cells. After pre-defined penetration times, cell lysates were separated under denaturing conditions on polyacrylamide gel (leftmost panel) or under native conditions on agarose gel (two rightmost panels). In both cases, samples were analysed by Western blot using anti-Dd antibodies. Control Dd samples contained 30 ng of protein, while control penton base (Pb) sample contained 10 ng of protein. **(C)** Stability of Dds in human serum. Dd samples concentrated by ultrafiltration in Microcon (Millipore) unit (5 µg each), were submitted to incubation in human serum (HS) at 4°C for 2 h (lane 4) and at 37°C for 15 min. or 2 h (lanes 5 and 6, respectively). Samples were separated on native gel and analyzed by Western blot using anti-Dd antibodies. The upper part shows the CBB (Commassie Brilliant blue)-stained gel with residual proteins after the transfer, and the lower part shows the developed Western blot. Lanes 1 and 7 correspond to serum-free Dd samples, non treated or incubated for 2 h at 37°C, respectively. Lanes 2 and 3 correspond to human serum incubated for 2 h at 4 and 37°C, respectively.

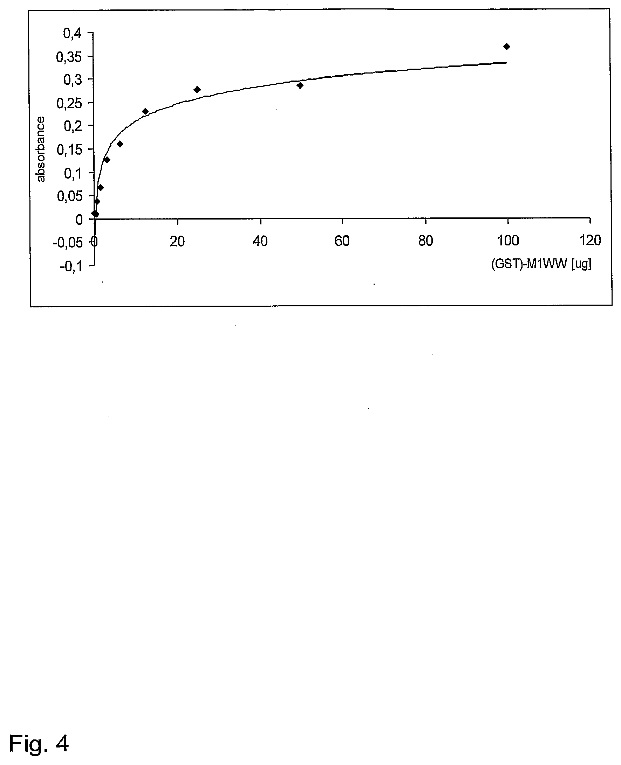

**Figure 4** presents a modified ELISA assay demonstrating an interaction of the Dd with fusion protein GST-M1WW, prepared as described in Example III (see further).

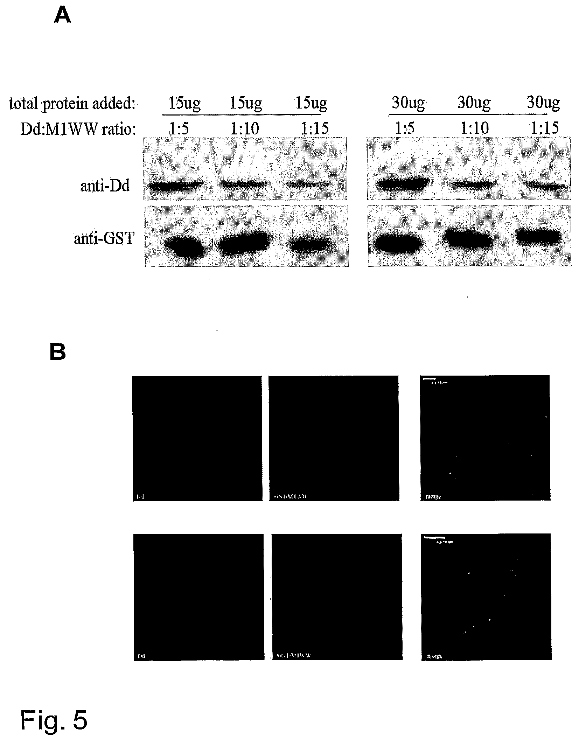

**Figure 5** presents internalization of the Dd/ GST-M1WW complex. Dd/ GST-M1WW complexes obtained by combining the components at different ratios were introduced into HeLa cells. The efficacy of internalization was analysed using anti-Dd and anti-GST antibodies: **(A)** by Western blot; **(B)** by confocal microscopy. Shown are a green signal of the Dd (white/grey in black and white images) and a red signal of GST-M1WW (white/grey in black and white images).

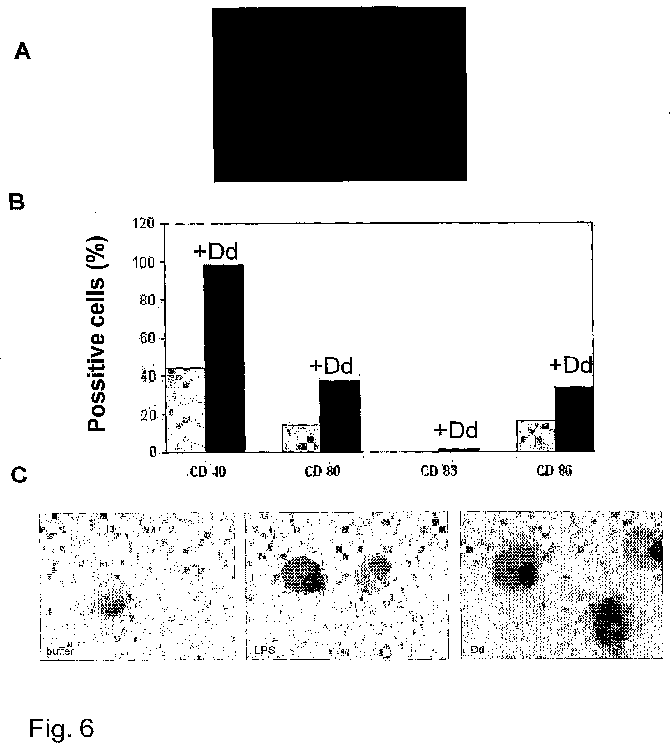

**Figure 6** presents activation of myeloid dendritic cells (MDCs) by Dds. Dd-treated MDCs were analysed by: **(A)** confocal microscopy using anti-Dd antibody - green cytoplasmic signals (white/grey in black and white images); **(B)** by flow cytometry by testing the activation of surface markers: CD40, CD80, CD83 and CD86; **(C)** by optical microscopy while observing morphological changes. Control cells were treated with buffer or lipopolysaccharide (LPS).

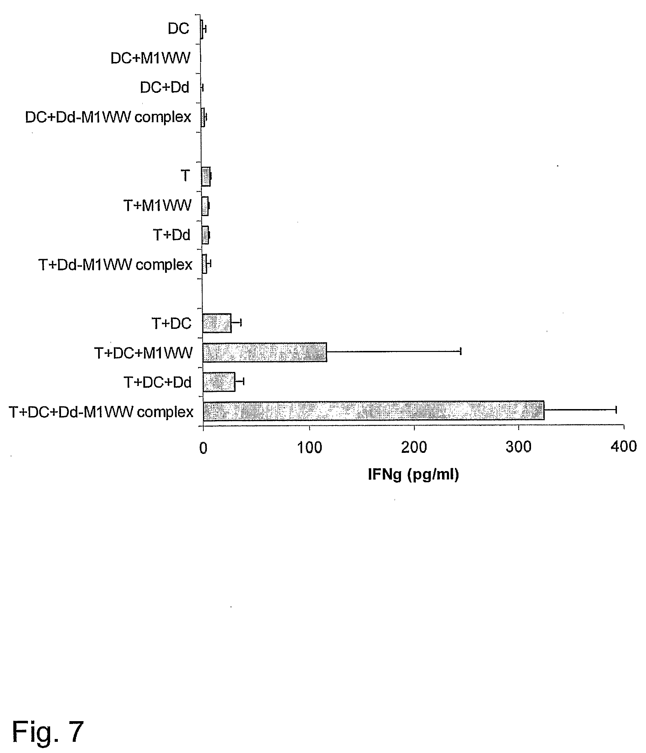

**Figure 7** shows a cross-presentation of M1 peptide to specific T lymphocytes. Dendritic cells transduced with Dd or M1WW antigen alone or with the Dd-M1WW complex were incubated with T lymphocytes specific to the immunodominant epitope of the M1 protein. After 24 hours of co-culture, supernatants were collected, and interferon-gamma (IFNγ) content was measured by flow cytometry. The obtained results suggest that the Dd-M1WW complex was efficiently taken up and processed by the DC, leading to presentation of peptides formed from M1 protein to lymphocytes sensitized to M1.

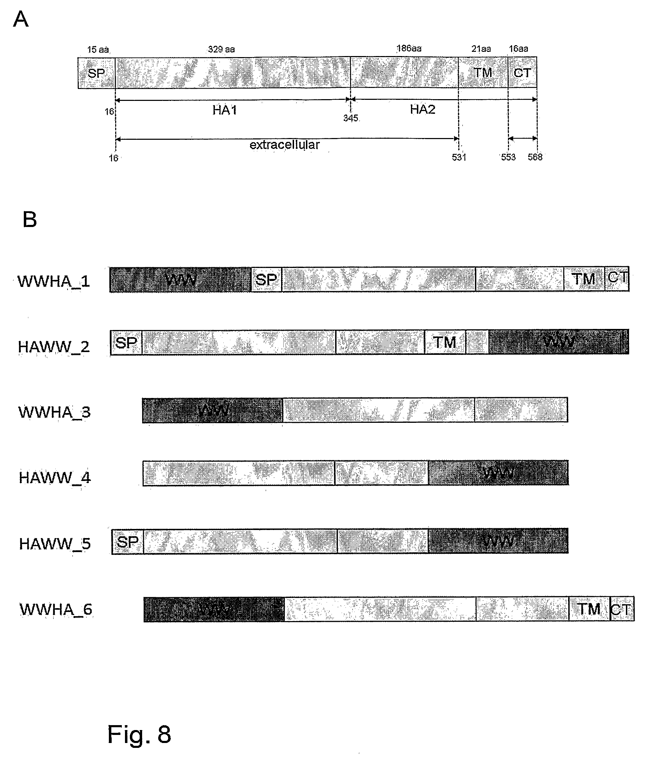

**Figure 8** shows **(A)** a schematic presentation of the protein sequence of hemagglutinin and **(B) -** different variants HAWW and WWHA fusion proteins. SP - signalling peptide, TM - transmembrane domain, CT - cytoplasmic tail, WW - WW 1,2,3 domain from Rsp5 protein.

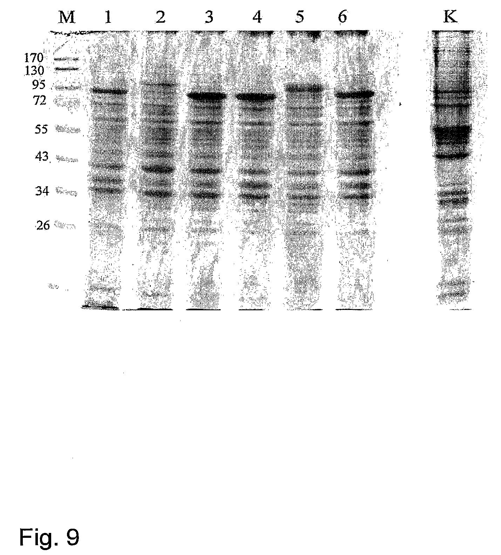

**Figure 9** shows an analysis of production of recombinant HAWW and WWHA proteins. Insect cells were infected by recombinant baculoviruses, collected 48 hours after infection and separated on a denaturing gel stained with CBB. The following were applied to the successive lanes: 1 - WWHA_1; 2 - HAWW_2; 3 - WWHA_3; 4 - HAWW_4; 5 - HAWW_5; 6 - WWHA_6; K - Control cells not expressing HAWW.

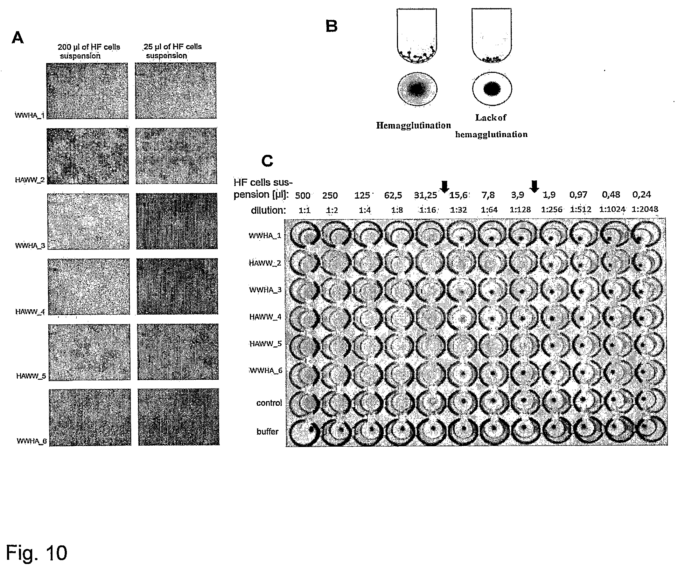

**Figure 10** presents functional analysis of HA variants. **(A)** Hemadsorption assay. Protein expressing HF cells (200 µl or 25 µl) were incubated with suspension of hens red blood cells and the hemadsorption was observed under light microscope. Nonexpressing cells and dilution buffer were used as controls. (B) Schematic representation of hemagglutination visible as erythrocyte spread across the well bottom, in contrast to non-hemagglutinizing erythrocytes which sediment on well bottom; (C) Hemagglutination obtained for respective HAWW and WWHA protein variants. HF cells expressing the recombinant proteins were suspended in hypotonic buffer (20 mM Tris, pH 7.5, 50 mM NaCl, 2 mM EDTA), and lysed by freezing/thawing (3 rounds). Lysates (corresponding to 10⁶ HF cells) were placed in wells of a 96-well U-bottom plate and serially diluted in hypotonic buffer. 50 µl of 1% suspension of hen erythrocytes in PBS were added to each well and the plate was incubated for 1 h at room temperature. Cells that did not produce the recombinant protein (control) and the hypotonic buffer (buffer) were used as controls. Hemagglutination was assessed visually and photographed. Hemagglutination was observed for all cellular clones (including control cells) at cell suspension dilutions of 1:1 to 1:16. In case of HAWW_2 and HAWW_5 clones, hemagglutination was additionally observed at much higher dilutions from 1:32 to 1:128 (marked by arrows).

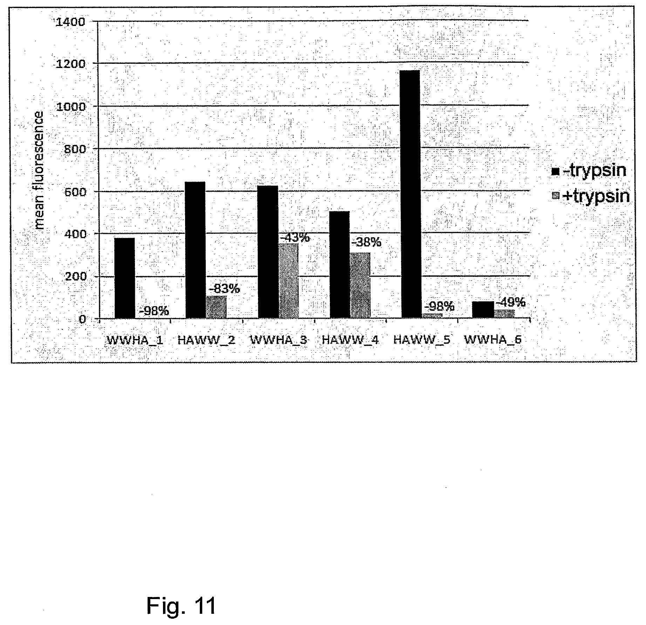

**Figure 11** includes a graph presenting expression of particular variants of HAWW proteins on the surface of HF cells (black bars) and expression of the same proteins after treating the cells with trypsin (grey bars), measured by flow cytometry. Vertical axis marks the fluorescence level. Relative change in fluorescence after the trypsin treatment of cells is expressed above bars in percentage values.

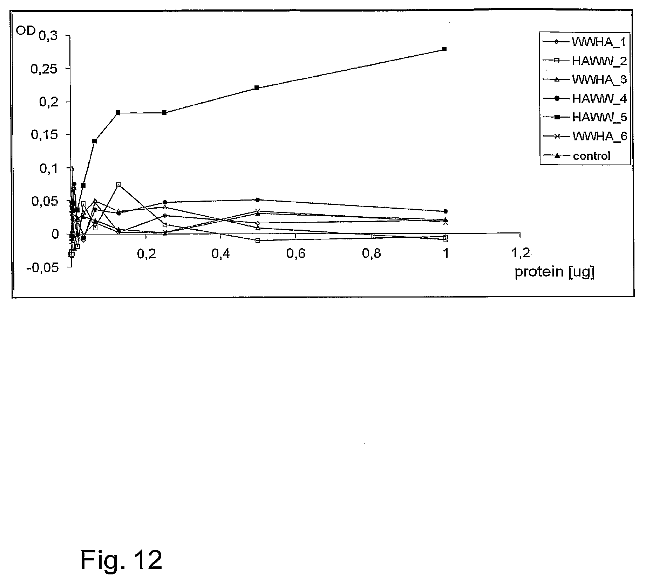

**Figure 12** presents an ELISA assessment of capability of HF cells, expressing different variants of HAWW and WWHA proteins, to interact with the dodecahedra. A plate was coated with dodecahedron and incubated with serial dilutions of cell suspensions. Relative amount of cells tethered to the dodecahedra were detected by anti-HA antibody in a colorimetric assay.

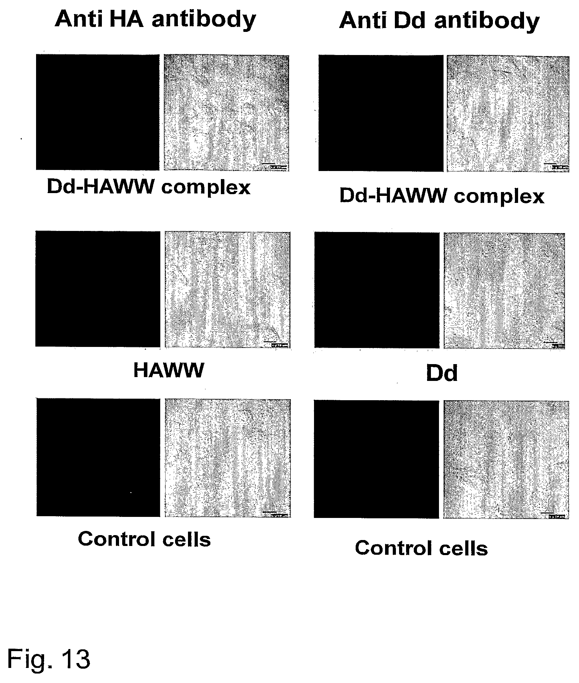

**Figure 13** presents the introduction of HA antigen into human cells. The confocal fluorescence microscopic image shows HeLa cells after internalization of the Dd-HAWW complex (top row). Hemagglutinin or dodecahedron are marked red (white/grey signal in black and white images). Cell nuclei were stained blue using DAPI (marked grey in black-and-white images). Additionally, light transmission images, called Nomarski contrast, are shown (rightmost columns).

Presented below are example embodiments of the invention described above, which invention it its broadest sense is as defined in the independent claims.

### Examples

The authors have constructed virus-like particles, denoted herein as Dd, from adenoviral protein responsible for endocytosis, and used the Dd as a vector for transporting influenza antigens. The vector is characterized by numerous interesting properties. Dd is very efficient in cell penetration, is stable at temperatures of up to 50°C, and may be stored frozen and/or lyophilized (Zochowska et al., 2009). The size of the Dd is approximately 40 nm, which suggests that in contrast to larger VLPs (above 500 nm) Dd would be capable of reaching not only the dendritic cells within the injection area, but also to dendritic cells residing in distant lymph nodes. In addition, the antigen is tethered via a protein linker obtained from yeast protein, which should eliminate the risk of possible autoimmune reaction. Besides, the yield of recombinant Dd produced in a baculovirus system is comparable to the most efficient bacterial systems (ca. 10 mg Dd per 100 ml of insect cell culture (Zochowska et al., 2009; Song et al., 2008).

Dd with the tethered antigenic proteins M1 and HA penetrates into the cell interior. Intracellular penetration of the external HA antigen is known to result in an intense induction of the production of neutralizing antibodies, protecting the system from influenza virus infection (Samji, 2009). In case of the internal influenza antigen, M1, internalization of antigen by dendritic cells causes cell maturation, resulting in effective presentation of the peptide to CD8+ T lymphocytes. The reaction of T lymphocytes triggered by this complex should provide appropriate protection against development of acute medical condition. In addition, since antigens recognized by T lymphocytes are obtained from internal viral proteins, which are more conserved than the external proteins, the embodiment delivers a vaccine characterized by a wide spectrum of activity against different influenza virus strains.

Biotechnological solutions used in this invention will permit further addition of multiple epitopes to prevent immunological escape of the virus and to supplement the vaccine with additional antigens such as toll-like receptor (TLR) ligands and ligands recognized by CD4+ T lymphocytes. Importantly, the use of a symmetric polyvalent vector as a vaccine platform enhances presentation of antigens.

In conclusion, the Applicants have shown that attachment of influenza virus antigens to adenovirus dodecahedron did not inhibit the excellent penetration efficiency of the vector, shown by high antigen transfer into the dendritic cells. In addition, the vaccine in the form of a non-covalent complex of a vector and two different antigens led to activation of dendritic cells and would most probably trigger production of neutralizing antibodies. This the new vaccine, by efficient uptake, presentation of the antigen by the DCs, followed by induction of virus-neutralizing antibodies and possibly, cellular response, would ensure efficient protection against influenza virus infection.

The dodecahedron of Applicants' is derived from serotype 3, belonging to subgroup B with tropism directed towards respiratory and visual systems. Since amino acid sequences of penton bases and fibre bases involved in the formation of the penton, and thus the dodecahedron, are highly conserved, it is possible to produce, by means of genetic manipulation, chimeric Dds with altered tissue tropism. An example can include the Dd derived from Ad3, which, owing to attachment of Ad41 short fibre protein will attain affinity to human gastrointestinal system.

Our own research regarding the adenovirus dodecahedron was related to expression, purification and characterization of dodecahedra, as well as the use of said dodecahedra for intracellular delivery of therapeutic agents by employing a specific linker. The research included:
- Obtaining high-grade dodecahedron preparations purified to homogeneity. Said preparations are free of proteins, nucleic acids and proteases derived from cells, where Dd is overproduced.
- Testing the vector stability. Factors such as pH, ionic strength and temperature were shown to affect the Dd integrity. Conditions allowing for storage, transfer and use of Dd preparations in various climates, including tropical ones, were developed.
- Constructing a Dd-specific linker derived from non-mammalian protein, allowing for non-covalent attaching of any protein or peptide to the Dd and introduction of said protein/peptide to the target cell, particularly for delivery of therapeutic agents into the tissues, particularly vaccines into animal tissues, particularly mammalian tissues.

In the method according to the invention, the Applicants have demonstrated that three WW domains derived from Rsp5 yeast protein act in tandems as universal adapters allowing any protein to be tethered to the dodecahedra and transferred to target cells. The efficiency of the system is remarkably high - about 10-20 million foreign protein molecules can be introduced into a single cell.

The virus-like particle vector according to the invention was found to allow transport of such therapeutic agents as proteins or peptides through cellular membranes (Garcel et al., 2006). The penton base protein, a building block of Dd, contains RGD motif that is known to interact with αv integrins, which are present at elevated levels in endothelial cells of newly formed vessels that supply blood to the tumour tissue (Chen, 2006). Dd is composed of 12 copies of pentameric penton base and therefore, a Dd containing 60 RGD motifs is a very specific partner of αv integrins, while being highly capable of penetrating cells due to its endosmolytic activity and affinity to heparan sulfates. This suggests that e.g. the use of Dds for the delivery of anticancer therapeutic agents also means that these agents would specifically target the neoplastic blood vessels supplying the tumours.

### Use of Dd as a vector for the transport of protein/peptide-based therapeutic agents

Delivery of proteins using Dd is a very efficient process in which 100% of cells are transduced (Fig. 1). Efficient introduction into the cytoplasm of multiple copies of large multimeric proteins, such as monoclonal antibodies recognizing the dodecahedra, was shown to be possible (Fender et al., 2003), suggesting high efficiency of transduction with the aid of Dds.

### Example: Delivery of influenza virus antigens - M1 and HA proteins

Two antigens were selected for the construction of a novel influenza vaccine: one of the genetically most stable influenza proteins, i.e. the internal matrix protein 1 (M1) and the external protein, hemagglutinin (HA), with the purpose of delivering these antigens on a platform formed by adenovirus dodecahedron particles built of adenoviral penton base proteins. The applicant has proposed the use of WW domains for attaching the influenza antigens to the Dd. Fusion protein antigens of the invention include multiple copies of the M1 or HA protein, or of fragments thereof, genetically fused to a linker protein WW, derived from the yeast protein Rsp5.

The antigen protein M1WW was obtained by expression in *E. coli*, while the antigen protein HAWW was obtained by expression in the baculovirus eukaryotic system. Interaction of the Dd vector with antigens was confirmed by a modified ELISA assay. Successful internalization of the generated complex in HeLa cells was observed by biochemical methods and by confocal microscopy. Additionally, internalization of the M1 protein-carrying dodecahedron resulted in activation of human dendritic cells. As a result of this research, the use of a dodecahedron carrying influenza virus proteins was proposed as influenza vaccine. It was demonstrated that such vaccine model is feasible and that Dd may act as a multivalent platform for the delivery of foreign antigens to human cells.

The method according to the invention to tether the M1 or HA protein to the Dd vector made use of a linker protein WW interacting with the dodecahedron via Dd PPxY motifs. Genetic fusion of the influenza antigens with the WW linker allows tethering the antigens to the vector in a polyvalent manner. This invention describes the synthesis, isolation and construction of a novel influenza vaccine, as well as functional analyses of said vaccine.

### Activation of myeloid dendritic cells (MDCs) by the dodecahedron.

Dendritic cells (DCs) are known to be the most versatile antigen-presenting cells. The main functions of the DCs are to capture the antigen, transfer it to the lymph nodes and present to T lymphocytes, which leads to initiation of immune response to that particular antigen. The Applicants have demonstrated that human dendritic cells are activated by the dodecahedral vector itself, which should allow for an enhancement of the response of T lymphocytes (see example V).

### Internalization of the vaccine (Dd in complex with M1WW antigen and Dd in complex with HAWW antigen)

The complex of Dd with antigens fused to WW linker according to the invention were obtained by mixing the vector with the antigen. The complex was introduced to HeLa cells. The efficacy of internalization was assessed by the immunochemical Western blot method. Simultaneously, vaccine internalization was observed by confocal microscopy. HeLa cells were incubated with a complex of Dd with antigens fused to WW linker or with Dd alone. Vaccine localization was examined after 1 hour of internalization. The complexes were efficiently internalized in HeLa cells, being uniformly distributed in the cytoplasm.

The method according to the invention made use of a WW linker, which was shown to have specific affinity to the dodecahedral vector. In this patent, WW domains linker derived from yeast protein is being used. In the method according to the invention, the Dd is obtained in a baculovirus system as a recombinant protein (rDd) with an exceptionally high yield, which amounts to 10 mg rDd per 100 ml of insect cell suspension. This expression yield is comparable to that achieved in the most efficient bacterial systems. Uptill now, rDd has been purified only by means of ultracentrifugation in sucrose gradient. This stage allows for elimination of low- and medium-molecular cellular proteins, but not of nucleic acids, which are most probably attached to the Dd surface. The planned therapeutic application of rDd required more homogeneous and better purified preparation, which was achieved by a 2-stage protein purification process. Thus, following preliminary purification of rDd in sucrose gradient, ion exchange chromatography was used. A pure rDd fraction (purity above 95%) was obtained, as determined in the analysis of the preparation by polyacrylamide gel electrophoresis and by electron microscopy.

The biochemical and biophysical examinations performed (electron microscopy, native agarose gel and denaturing polyacrylamide gel electrophoresis, as well as dynamic light scattering (DLS) measurements) showed that rDd is stable at temperatures of up to 40°C in a wide range of pH values, and up to about 50°C at pH 7-8, at physiological NaCl concentrations (150 mM). Higher ionic strength conditions were found to significantly stabilize the rDd structure, preventing it from denaturation at temperatures up to 60°C. The vector particle maintains its integrity upon dialysis, after freezing, speed-vac drying and lyophilization in the presence of cryoprotectants. High stability of the vector facilitates both handling and storage of rDd (see example II).

In addition, rDd was found to maintain its integrity in conditions mimicking its application *in vivo,* i.e. in human serum at 37°C for at least 2 hours. The recombinant dodecahedra, purified according to the procedure developed by the Applicants, efficiently penetrate human tumour cells *in vitro*. The above findings allow rDd to be used as a vector in various applications and at different climate conditions.

The properties of the Dd described above imply a possibility to use this vector for delivery of therapeutic agents to human tissues. Presented below are example embodiments of the invention, which in its broadest sense is as defined in the independent claims.

### Example I

### Adenovirus dodecahedron preparation method

The planned therapeutic use of rDd required a homogeneous preparation, which was achieved by a 2-stage protein purification process. Following preliminary purification of rDd in sucrose density gradient, low-pressure ion exchange chromatography was used. Fraction of pure rDds, another fraction containing free pentameric bases (Dd building blocks) and a third fraction containing nucleic acids were obtained, suggesting low homogeneity of Dd obtained in one-step purification.

At the beginning, recombinant baculovirus containing a full-length human Ad3 penton base protein gene was obtained. Amplification of the recombinant virus carrying the penton base gene was performed in a monolayer culture of *Spodoptera frugiperda* cells (Sf21). Cells were maintained in TC100 medium supplemented with 5% fetal calf serum (Invitrogen). Dds were expressed in *Trichoplatsia ni* (T. ni, in. HF) grown in suspension in Express Five SFM (Invitrogen) with gentamycin (50 mg/l) and amphotericin B (0.25 mg/l). HF cells were infected with the recombinant baculovirus at multiplicity of infection (MOI) of 4 pfu/cell. After 48 hours from infection, cells were collected and lysed by three rounds of freezing and thawing. Supernatant obtained after lysate centrifugation was applied to 15-40% sucrose density gradients (Fender et al., 1997). The ultracentrifuged preparation of VLPs recovered in 30-40% sucrose was contaminated with other proteins and nucleic acids. Final Dd purification was achieved by means of ion exchange chromatography, resulting in a homogeneous dodecahedra fraction. Oligomeric status of the particles was analysed on native agarose gels and by electron microscopy, and the purity of the obtained preparations was assessed on polyacrylamide gels.

### Example II

### Adenovirus dodecahedra stability studies

The stability and solubility of the purified Dd particles was tested. To this end, purified rDds were dialysed against different buffers (with three changes of each) and incubated at 30 or 37°C. After incubation, samples were centrifuged and oligomeric status of proteins in the supernatant was determined by agarose gel electrophoresis.

Dd particles were dissolved at 4°C and pH of 4 to 10.9 in the presence of 150 mM NaCl. In the absence of NaCl, Dds are not retained in the solution, disappearing from the supernatant during centrifugation, which leads to a conclusion that NaCl at physiological concentration protects Dd from denaturation.

In order to assess the Dd resistance against thermal denaturation, Dynamic Light Scattering (DLS) technique was used, as it allows monitoring of protein denaturation or decomposition. Protein samples (0.2 mg/ml) were dialysed and filtered (0.45 µm pore filters) against appropriate buffers. Samples were placed in a cuvette (45 µl, Greiner, Frickenhausen, Germany). Automated particle size measurements were collected using ZS Nano Zetasizer (Malvern, Worcestershire, UK). The temperature changed by 2°C every 2 min in the range of 12 to 65°C. Data were assessed using a cumulative method (Fig. 2A).

Within the pH range of 4-9, the particle size was stable up to 40°C. Above this temperature, the particle size increased exponentially, indicating denaturation. At pH 4-5, Dd denaturation/aggregation started at temperature lower by about 10°C than at pH 7-8. At pH 9 (CAPS buffer) and 10 (carbonate buffer) only small changes in particle size were observed. Native gel analysis of proteins showed that while at pH 10 Dd dissociates to free bases, at pH 9 (CAPS buffer) the protein disappears, probably as a result of aggregate formation. It must be noted that CAPS is an organic buffer that may have a propensity to interact with hydrophobic patches on the protein surface, which can induce protein aggregation (Fig. 2B).

Addition of 750 mM NaCl to PBS caused a positive shift in the melting point (Tₘ) of Dd, suggesting structure stabilization (Fig. 2C). However, the most significant increase in Tₘ values was caused by addition ofammonium sulphate (positive shift of about 12°C, Fig. 2C). The studies showed that Dd structure was preserved upon dialysis, freezing and drying in the speed-vac in the presence of 150 mM ammonium sulphate. Presence of a cryoprotectant is required for the Dd structure to be maintained upon lyophilization (Fig. 3A).

The stability of the vector in the cell culture was tested in HeLa cells at different time points following Dd administration in the following manner. Purified Dds (4 µg/100 µl, 10.8 nM) in FCS (fetal calf serum)-free EMEM medium were added to HeLa cells (2x10⁴ cells) and the cells were placed in the incubator. Three hours after Dd application, the medium was enriched with FBS to the final concentration of 10%. Cells were collected at indicated time points (Fig. 3B) and lysed in hypotonic buffer. Samples containing half of the cell extracts were resolved on polyacrylamide denaturing gel (SDS-PAGE), while the other half was resolved on native agarose gels. Western blot with anti-Dd antibodies was performed in both cases. It appeared that the amount of intracellular Dd increased until 32 h post-transduction, with noticeable Pb proteolysis resulting in only a fraction of base protein remaining after 4 days (Fig. 3B, leftmost panel). Native agarose gel analysis showed that 96 h post penetration, the majority of intracellular material migrated between Dd and Pb, suggesting the removal of external loops from Dd with particle integrity maintained.

### Dd stability upon incubation in human serum

Dd samples (5 µg each), concentrated by ultrafiltration in Microcon (Millipore) unit were submitted to incubation in human serum (HS) at 4°C for 2 h and at 37°C for 15 min. or 2 h. Dd maintains its integrity in conditions mimicking its application *in vivo*, i.e. in human serum at 37°C for at least 2 hours (Fig. 3C).

The above results suggest that Dd may be conveniently stored and transported, and that it may potentially be used for therapeutic purposes in different climate conditions.

### Example III

### Preparation of adenovirus dodecahedron vector particle with WW protein and influenza virus antigen

### WW domains: cloning, expression and purification

WW1,2,3 proteins derived from yeast Rsp5 protein were initially placed in pY11 plasmid. The DNA fragment containing a WW1,2,3 (Rsp5) gene was synthesized by PCR with 5'-TTA **TTC GAA** CCG CCC TGG GGT CCT GA-3' i 5'-ATG ATG GCG CTA GGT **GTT CGA** GCT TAC GTA G-3' starters (sites recognized by *EcoRl* and *XhoI* enzymes marked in bold) and cloned into pGEX-4T-1 plasmid with the purpose of being fused with the GST protein. The correctness of cloning procedure was confirmed by DNA sequencing.

GST-WW fusion proteins were expressed in *E. coli* strain Rosetta 2 (DE3) (Novagen). The bacteria were grown at 37°C to optical density OD₆₀₀ₙₘ of about 0.6. Next, protein expression was induced with 0.4 mM (final concentration) IPTG for 4 h. Proteins were purified on Glutathion-Sepharose 4B matrix and analysed by SDS-PAGE and Western Blot.

### GST-M1WW protein cloning, expression and analysis

The gene coding for matrix protein 1 (M1) from influenza (Puerto Rico/8/34 H1N1), originally provided in pET15b was synthesized by PCR, using a 3'-terminal GGA C GGT CGA AAC TCT TCT GAG TAT **CCA ATT'** primer and a 5'-terminal GGA C GGT CGA AAC TCT TCT GAG TAT **CCA ATT'** primer (*EcoR1* restriction sites marked in bold) and cloned into pBlueScript plasmid. Next, the obtained M1 gene was ligated at *EcoR1* site of pGEX-4T-1 plasmid harbouring GST-WW fusion, between GST and WW domain genes. The correctness of the cloned protein GST-M1WW sequence was confirmed by DNA sequencing.

*E. coli* Rosetta 2 (DE3) strain (Novagen) was transformed with GST-M1WW-bearing plasmid. The cells were cultured at 37°C to OD₆₀₀ₙₘ of about 0.6. Next, protein expression was induced with 0.4 mM (final concentration) IPTG for 4 h. The recombinant fusion protein was insoluble upon expression in *E. coli.* It was extracted in the presence of 5 mM TCEP [Tris(2-carboxyethyl)phosphine] and 4.5% N-lauroylsarcosine in 50 mM Tris pH 8, 50 mM NaCl, 0.5 mM EDTA, 5% glycerol, using iFold protein refolding kit (Merck). To remove N-lauroylsarcosine, supernatants obtained from cell lysates by centrifugation (14,000×g for 30 min at 4°C) were dialysed against 50 mM Tris buffer, pH 7.5, containing 100 mM NaCl, 1 mM TCEP, 10% glycerol and 12.5 mM cyclodextrin. GST-M1WW fusion protein was purified on Glutathione-Sepharose 4Bcolumn (Amersham) with 20 mM reduced glutathione in 50 mM Tris pH 8.5, containing 100mM NaCl. GST tag was removed by 8 h hydrolysis with thrombin (Amersham) in the amount of 10 U of enzyme per 100 µg of protein. The GST was completely removed by polyacrylamide gel electrophoresis and the M1WW fusion, eluted by electrophoresis into a dialysis bag and precipitated with 4M (NH₄)₂SO₄ (final concentration). The protein precipitate was suspended in 50 mM Tris buffer, pH 7.5, containing 100 mM NaCl, 0.5 mM TCEP, 5% glycerol and 0.05% N-lauroylsarcosine. About 1 mg of purified protein was obtained from 500 ml of bacteria culture.

### HAWW protein cloning, expression and analysis

Since hemagglutinin - the external protein of the influenza virus capsid - is an oligomeric protein with a complicated processing, 6 different constructs were prepared, featuring deletions of different parts of hemagglutinin gene, as well as WW linker domains at N- or C-termini of HA protein (Fig. 8). Deletions of transmembrane and/or signalling sequence in individual HAWW and WWHA genes were introduced to reduce the risk that the proteins would be expressed in the membrane compartment of the insect cells.

WW1,2,3 derived from yeast Rsp5 protein, originally placed in pGEX-4T-1 plasmid, were re-cloned into pFastBacDual under the control of the p10 promoter. For this purpose, CTTC**GCTAGC**ATTCTTACCCCCTGGTTGG and CACGAGCAT**GCCGC**TACATATGGTCTAGCGATGATGG primers (*NheI* and *SphI* (*PaeI*) restriction sites marked in bold) were used to add restriction sites to the WW 1,2,3 (Rsp5) gene, and the gene was amplified by PCR. The insert was cloned into pFastBacDual plasmid between *NheI* and *SphI* restriction sites.

The gene coding for hemagglutinin HA0 of avian influenza A virus (A/swan/Poland/467/2006/H5N1), originally provided in pGemEasy, was cloned, depending on the construct, upstream of WW domains between *XhoI* and *NheI* sites, or downstream of WW domains between *SphI* and *NdeI* sites. Individual inserts were prepared by PCR using primer pairs listed in Table 1. Next, the PCR products were digested with *XhoI* / *NheI* or *SphI* / *NdeI* enzymes, respectively, and ligated to linearized pFastBacDual plasmid carrying the WW linker gene. The correctness of cloning procedure was confirmed by DNA sequencing. The DH 10 Bac (*E. coli*) cells were transformed by DNA isolated from six recombinant plasmids containing WWHA and HAWW genes. After recombinant bacmid DNAs were obtained, transfection to *Spodoptera frugiperda* Sf21 insect cells was carried out, using standard procedures as described in Bac-to-Bac Baculovirus Expression System Manual (Invitrogen). Amplification of recombinant baculoviruses was performed in a monolayer culture of Sf21 cells in TC100 medium containing 5% FCS (Invitrogen). Baculovirus titre was determined by plaque assay. Individual variants of HAWW and WWHA proteins were expressed in *Trichoplatsia ni* (*T. ni*, in. HF cells) grown in suspension in Express Five SFM (Invitrogen) with gentamycin (50 mg/l) and amphotericin B (0.25 mg/l) at 27°C with shaking (100 rpm). HF cells were infected with viral suspension at MOI value of 5. Optimum expression time for the recombinant proteins was determined to be about 48 hours. All HAWW and WWHA protein variants were expressed in insect cells. The recombinant proteins migrate in denaturing gels in a manner that is different from what could be expected from their molecular weights (Fig. 9). This is probably due to glycosylation of hemagglutinin.

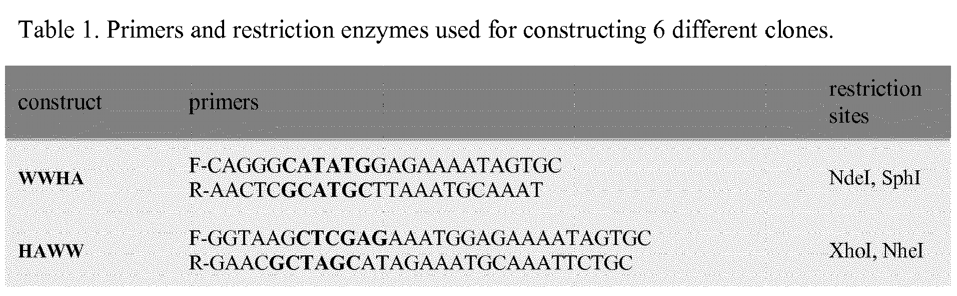

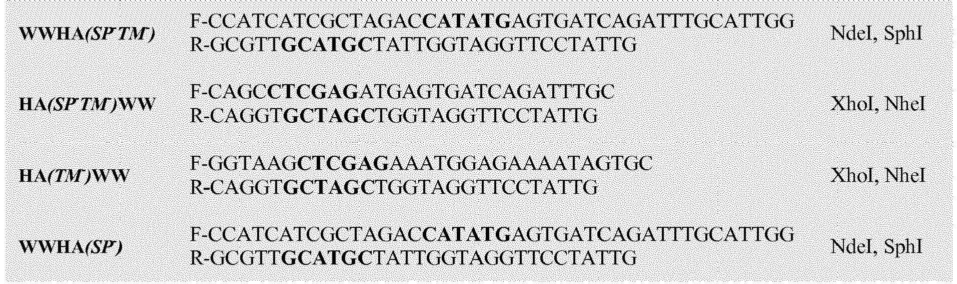

Solubility of the obtained proteins was tested on extracts of expressing cells obtained by sonication, rounds of freezing/thawing in hypotonic buffer (20 mM Tris, pH 7.5, 50 mM NaCl, 2 mM EDTA), as well as by incubation in commercial cell lysis reagents, such as Proteo Jet (Fermentas), Bug Buster, PopCulture, Cytobuster (all from Novagen). Protein expressed by HAWW_5 clone was found to be partially soluble by all the techniques listed above. WWHA_3 and HAWW_4 proteins could be extracted, to a small degree, by sonication and freezing/thawing. In addition, WWHA_3 could also be extraced by Proteo Jet (Fermentas). All proteins obtained in the partially soluble form were variants devoid of the transmembrane domain (TM), while the remaining proteins containing the TM (WWHA_1, WWHA_2 i WWHA_6) remained in the insoluble fraction.

In order to identify the recombinant proteins expressed on the surface of viable insect cells, fluorescence measurements were performed for non-permeabilized live cell,s incubated with fluorescently labelled anti-HA antibody. Two series of clones (1-6) were studies, with one that was preliminarly proteolysed (Fig. 11). Suspension of HF cells corresponding to 500 µl culture was incubated in the presence of 1 µg of trypsin (Sigma) in the volume of 500 µl for 5 minutes on ice. Protease inhibitor (Complete) was added to stop the proteolysis. Afterwards, the cells were incubated with primary anti-HA antibody (1:100 in PBS, 1 h, 37°C), and next with Texas-Red-conjugated secondary antibody (1:250 in PBS, 1 h, 37°C). Finally, cell suspensions in PBS were studied using FACSCalibur cytometer (Becton Dickinson), and the results were analysed using CellQuestPro software (Becton Dickinson). All HAWW and WWHA proteins were detected on the surface of insect cells, HAWW_5 protein being expressed in largest amounts and at the same time being most sensitive to proteolysis (i.e. most accessible). Incubation of cells in the presence of trypsin has also reduced the signals of WWHA_1 and HAWW_2 proteins, which possibly shows the effect of signalling sequence (SP) comprised in these proteins (see Fig. 1) on cell surface expression.

Hemadsorption and hemagglutination tests were performed to verify the activity of the recombinant hemagglutinin. HF cells were tested for their ability to adsorb hen's erythrocytes (haemadsorption test) and trigger their agglutination (hemagglutination test). Very apparent hemadsorption was observed for HAWW_2 and HAWW_5, suggesting external membrane expression of HA for these clones (Fig. 10A). Erythrocyte agglutination is a result of hemagglutinin's affinity to sialic acid, which is present at the erythrocyte surface. Hemagglutination, visible as uniform pink suspension in the microtiter plate, was observed for all variants of recombinant protein (as well as control cells, with no HA expression) when cells were diluted at 1:1 to 1:16 with appropriate buffer. However, HAWW_2 and HAWW_5 induced specific hemagglutination at higher dilutions of: 1:32, 1:64, 1:128 (Fig. 10C, indicated with arrows). Insect cells infected with empty baculovirus were not included as control in the experiment, since hen erythrocytes are not agglutinated by baculoviruses (Kumari et al., 2007). The obtained result suggests that only cells expressing HAWW_2 and HAWW_5 produced functional, and most probably correctly structured HA. Interestingly, both these proteins have WW domains at their C-termini, which might suggest a relationship between proper hemagglutinin structure and the position of genetically fused domain (WW linker). Nevertheless, taking in to consideration different solubility of the six HA variants, this result may also reflect different accessibility of HAWW/WWHA proteins for erythrocytes.

To sum up, these experiments characterizing the recombinant HAWW and WWHA proteins allowed for selection of one variant (HAWW_5) having structure and function appropriate for use in construction of influenza vaccine. The Applicants have developed a method for purification of this antigen. The Cytobuster lysate of HF cells expressing the HAWW_5 protein was centrifuged and the supernatant was diluted in 1:1 ratio with 20 mM MES buffer, pH 5.5. Thus prepared, the material was applied onto a heparin matrix column (1 ml, GE Healthcare) connected to BioLogic LP system, and purified with NaCl gradient. The heparin column allowed for removal of some protein contaminants, in particular a protein of molecular weight of ca. 60 kDa, characterized by strong affinity to concanavalin resin used in the next purification step. In parallel, HAWW_5 concentration was increased compared to the starting material. The next purification step made use of hemagglutinin's affinity to glycoproteins. Fractions containing HAWW_5 were combined and incubated with Concanavalin A-Sepharose resin (GE-Healthcare), for 1 hour at 4°C, gently shaking, in the presence of Ca²⁺ and Mn²⁺ ions (at 1 mM). After removal of the unbound fraction, the medium was incubated sequentially in 250 mM, 500 mM and 1M methyl-α-D-mannopyranoside solution (Sigma) in 20 mM MES buffer, pH 7.5 containing 100 mM NaCl.

The attachment of expressing cells to D was analyzed as follows. A 96- well plate (Nunc) was coated with the dodecahedra (ca. 2.5 µg per well) and, following saturation with 0.3% BSA in PBS, serial dilutions of insect cells expressing HAWW and WWHA proteins were added. The first well of each series contained 200 µl HF cells culture. The plate was incubated for 90 minutes with gentle shaking, and the unbound material was washed off. The amount of cells with HAWW and WWHA antigens bound to the vector was assessed spectrophotometrically by means of detection with anti-HA antibody. Fig. 12 shows a graph presenting the relationship between the amounts of HAWW and WWHA proteins carried by the dodecahedra and the absorbance readings. The obtained result shows that only the HAWW_5 clone attaches permanently to the dodecahedra. Thus, only HAWW_5 protein is expressed in amounts sufficient and in forms appropriate for generation of a complex with the vector. The above experiments confirmed the HAWW-Dd interactions.

### Example IV

### In vitro vaccine internalization, M1 antigen

Intracellular antigen delivery using the dodecahedron was studied by confocal microscopy. HeLa cells, prepared on coverslips were placed in wells of a 24-well plate, were incubated with the pre-formed Dd-GST-MIWW complex at 1:10 weight ratio, as well as with the Dd or the GST-M1WW fusion protein alone. After 1 h of incubation at 37°C, the coverslips containing cells were washed with sterile PBS, left to fix in cold methanol for 5 min., and blocked with 5% BSA in PBS for 30 min. at 37°C. Coverslips were rinsed with PBS and incubated with appropriate antibodies. Polyclonal rabbit anti-Dd antibodies at 1:1000 dilution and mouse monoclonal anti-GST antibodies (Sigma) at 1:250 dilution were used for GST-M1WW antigen detection, each for 1 hour at 37°C. Texas Red-labeled anti-rabbit antibodies and FITC-labelled anti-mouse antibodies were used as secondary antibodies at 1:250 dilution. Nuclei were stained with DAPI (1 µg/ml). Before observation, preparations were again washed with PBS, and then the coverslips were mounted on slides. Preparations were observed with laser scanning confocal microscopy, and the images were processed using the EZ-C1 Free Viewer software (Nikon). The complex was efficiently internalized in HeLa cells and was uniformly distributed in the cytoplasm (Fig. 5B).

### Internalization of HAWW_5 in complex with Dd in HeLa cells

The experiments characterizing the recombinant HAWW and WWHA proteins described above allowed for selection of one variant having appropriate structure and function. This protein was used for generation of vector-HA antigen complexes. HAWW_5-expressing cells were lysed with Cytobuster (Novagen) used in the ratio of 150 µl per 1x10⁶ cells, for 5 min. at room temperature, and the resultant lysate was incubated with the dodecahedra to obtain the Dd-HAWW_5 complex. Thus prepared, the material was added to HeLa cells grown on coverslips. After 60 minutes of internalization, the cells were washed with sterile PBS, permeabilized and fixed by addition of cold methanol. After another wash, the cells were incubated in turn with 5% BSA in PBS, and then with primary rabbit polyclonal anti-Dd antibody at 1:1000 dilution or anti-HA antibody at 1:100, each for 1 hour at 37°C. Texas Red-labelled anti-rabbit antibody was used at 1:250 dilution as the secondary antibody. Cell nuclei were stained with DAPI (1 µg/ml, Pierce). Thus prepared, the coverslips were attached to slides using Mowiol (Sigma), which simultaneously acted as a fluorescence-stabilizing medium. Presentation of co-localization of vector and antigen by using different secondary antibodies was impossible due to the rabbit origin of both the anti-Dd and anti-HA antibodies. However, a strong signal obtained with the anti-HA antibody in cells incubated in the presence of the complex compared to no signal in control cells incubated with the same antibody is a confirmation of the antigen being introduced to cells by the dodecahedric vector (Fig. 13).

### Example V

### Activation of myeloid dendritic cells (MDCs) by the dodecahedron

Internalization of Dds into the MDCs was confirmed by confocal microscopy (Fig. 6A). Simultaneously, Dd-induced expression of activation markers: CD40, CD80, CD83 and CD86, on the surface of the MDCs, was monitored. At 24 hours, application of Dd resulted in induction of three of the four markers (Fig. 6B). Simultaneous observation by optical microscopy revealed morphological changes characteristic of mature MDCs (Fig. 6C). These results confirm that dodecahedra alone stimulate maturation of dendritic cells.

### Example VI

### Analysis of the kinetics of internalization of the Dd-antigen complex by myeloid dendritic cells

The Dd-WW 1,2,3 (Rsp5) complex was generated at room temperature by 1-hour incubation of 20 µg of Dd with 60 µg of WW1,2,3 Rsp5 (fused to GST). Aliquots of the complex solutions (100 µl) were added to four cell samples, each containing 3.8 x 10⁵ MDC in 1ml of PBS. Simultaneously, MDCs were incubated with 20 µg of Dd alone. Cells were harvested after 30 min., 60 min., 90 min. and 120 min. of incubation, washed with sterile PBS and fixed with cold methanol (-20°C, 30 min). Rabbit polyclonal anti-Dd serum at 1:500 dilution and mouse monoclonal anti-GST antibodies (Sigma Aldrich, St. Louis, MO, USA) at 1:250 ratio were used for complex detection, each for 1 hour at 37°C. Secondary anti-rabbit-PE and anti-mouse-FITC antibodies (both obtained from Jackson ImmunoResearch, West Grove, PA, USA) were used at 1:250 ratio for 30 min. on ice. A portion of cells with no proteins added was incubated with antibodies for background signal determination. Kinetic analysis of complex internalization by the dendritic cells was performed by flow cytometry using a FACScan (Becton Dickinson) cytometer, and the results were analysed using Cellquestpro software.

### Example VII

### Generation of anti influenza specific CD8+ T lymphocytes and cross-presentation of M1 by myeloid dendritic cells

T lymphocytes obtained after sampling from healthy HLA-A*0201 donors (who gave an informed consent), were specifically primed to the immunodominant peptide 58-66 of the M1 protein (Manches et al., 2008). Peripheral blood mononuclear cells were purified by gradient density centrifugation and frozen. Myeloid dendritic cells (MDCs) were generated from purified monocytes and matured by a 2-day culture in the presence of CD40L (Alexis Biochemicals, Lausanne, Switzerland). Mature MDC were incubated for 3 h with the M1 58-66 peptide (GILGGFVFTL, 1µM, Neosystem, Strasbourg, France) in the presence of β2-microglobulin. CD8+ T lymphocytes were enriched from thawed peripheral blood mononuclear cells (PBMCs) (StemSep human CD8+ T cell enrichment kit, StemCell Technologies, Grenoble, France) and cultured (1 x 10⁶ T cells/ml) with MDCs previously incubated with the M1 58-66 peptide (T:MDC = 10:1) in RPMI medium supplemented by 10% FCS. Cells were diluted at days 6 and 10 with medium containing IL2 (interleukin 2) (final concentration 20U/ml) and IL15 (Interleukin 15) (25ug/ml). At day 12, T lymphocytes directed against anti M1 58-66 peptide were quantified by tetramer labelling (Beckman Coulter Inc., Fullerton, USA) and frozen. The preparations contained more than 50% M1 specific CD8+ Tlymphocytes.

Lymphocyte secretion of interferon gamma (IFNγ) is a measure of presentation of antigen by the dendritic cells. For presentation assay, MDCs were thawed and incubated for 3 h with M1WW antigen (1µg/ml), or with Dd (0.5 µg/ml), or with the Dd-M1WW complex. Cultures in 96-well round-bottom plates were incubated in RPMI medium containing 10% FCS. MDC (10,000 cells/well) were mixed with M1-specific CD8+ T lymphocytes (25,000 cells per well) and cultured in three identical wells for 24 hours. Culture supernatants were then harvested and their IFNγ content was measured by flow cytometry (Becton Dickinson).

### Results

### Uptake of Dd-M1WW vaccine into myeloid dendritic cells and presentation of M1 antigen to specific lymphocytes T

Following internalization of the vaccine complex Dd-M1WW by the MDCs, M1-specific lymphocytes T were used for the assessment of antigen presentation efficacy. M1-specific lymphocytes T, i.e. lymphocytes that were in contact with the dendritic cells pre-incubated with the Dd-M1WW complex, released IFNγ, showing efficient uptake, processing and presentation of the antigen by the MDCs (Fig. 6). MDCs incubated with M1WW protein alone also induced IFNγ secretion in lymphocytes T; however, the secretion was approximately three times lower than when M1WW was in complex with Dd, which shows that Dd exerts an enhancing effect when used as a vector for delivery of antigens to dendritic cells.

Also in case of the other influenza antigen, hemagglutinin, a complex with the vector was obtained. Using confocal microscopy, the applicants have demonstrated efficient internalization of Dd - HAWW_5 complex into human cells in an *in vitro* culture. This proves the stability of the formed complexes and confirms their utility for the vaccine construction.

### Bibliography

Chen X. Multimodality imaging of tumor integrin alphavbeta3 expression. Mini Rev Med Chem. 2006 Feb;6(2):227-34.

Fender P., Ruigrok R.W., Gout E., Buffet S., Chroboczek J. (1997) Adenovirus dodecahedron, a new vector for human gene transfer. Nat. 15 (1), 52- 56.

Fender P., Schoehn G., Foucaud-Gamen J., Gout E., Garcel A., Drouet E., Chroboczek J. (2003) Adenovirus dodecahedron allows large multimeric protein transduction in human cells. J. Viol. 77 (8) : 4960-4964.

Galinier R, Gout E, Lortat-Jacob H, Wood J, Chroboczek J. (2002) Adenovirus protein involved in virus internalization recruits ubiquitin-protein ligases. Biochemistry 41(48):14299-305.

Garcel A, Gout E, Timmins J, Chroboczek J, Fender P. Protein transduction into human cells by adenovirus dodecahedron using WW domains as universal adaptors. J Gene Med. 2006 Apr;8(4):524-31.

KoepfE.K., Petrassi H.M., Ratnaswamy G., HuffM.E., Sudol M., Kelly J.W. (1999). Characterization of the structure and function of WW domain variants: identification of a natively unfolded protein that folds upon ligand binding. Biochemistry 38(43):14338-51.

Kumari K, Gulati S, Smith DF, Gulati U, Cummings RD, Air GM. Receptor binding specificity of recent human H3N2 influenza viruses. Viol J. 2007 May 9;4:42-6.

Manches O, Lui G, Molens JP, Sotto JJ, Chapwot L, Plumas J. Whole lymphoma B cells allow efficient cross-presentation of antigens by dendritic cells. Cytotherapy 2008;10(6):642-9

Pawson T. and Scott J.D. (1997) Signaling through scaffold, anchoring, and adaptor proteins. Science 278(5346):2075-80.

Philipson L., Lonberg-Holm K., and Pettersson U. (1968) Virus-receptor interaction in an adenovirus system. J Viol. 2:1064-75.

Samji T. Influenza A: understanding the viral life cycle. Yale J Biol Med. 2009 Dec;82(4):153-9.

Song L, Nakaar V, Kavita U, Price A, Huleatt J, Tang J et al. Efficacious recombinant influenza vaccines produced by high yield bacterial expression: PLoS ONE. 2008;3(5):e2257.

Vives R.R., Lortat-Jacob H., Chroboczek J., Fender P. (2004) Heparan sulfate proteoglycan mediates the selective attachment and Internalization of serotype 3 human adenovirus dodecahedron. Virology 321:332-40.

Valentine, R. C. & Pereira, H. G. (1965). Antigens and structure of the adenovirus. J. molec. Biol. 13, 13.

Wickham T.J., Mathias P., Cheresh D.A., Nemerow G.R. (1993) Integrins alpha v beta 3 and alpha v beta 5 promote adenovirus Internalization but not virus attachment. Cell 73 (2) : 309-319.

Wohlfart C. (1988) J.Virol. 62, 2321-232.

Zochowska M, Paca A, Schoehn G, Andrieu JP, Chroboczek J, Dublet B, Szolajska E Adenovirus dodecahedron, as a drug delivery vector. PLoS ONE. 2009;4(5):e5569

## Claims

1. A virus-like particle vector **characterized in that** said vector is a recombinant adenovirus dodecahedron (Dd) particle consisting of pentons or penton base proteins from a mammalian adenovirus, particularly from an human adenovirus, which Dd carries non-covalently attached fusion proteins comprising multiple WW domains from fungal proteins, especially from yeast proteins, preferably from *S. cerevisiae* strain protein, preferably from Rsp5 protein, genetically fused to a protein or peptide therapeutic substance comprising an antigen, preferably an influenza virus antigen, and wherein said WW domains interact with PPxY (proline-proline-any amino acid-tyrosine) motifs of the Dd.

2. Particle according to Claim 1, **characterized in that** three WW domains derived from Rsp5 protein act in tandem as universal adapters allowing multiple copies of any protein or peptide to be tethered to the dodecahedra.

3. Particle according to Claim 1, wherein the fusion proteins have the WW domains at their C-terminus.

4. Particle according to Claim 1, **characterised in that** the antigen protein is influenza virus matrix protein M1 or hemagglutinin HA, or fragments thereof, genetically fused to the WW linker protein.

5. Method for generating a virus like particle vector described in claims 1 to 4, **characterized in that** the recombinant adenovirus dodecahedron particle consisting of pentons or penton base proteins from a mammalian, particularly human, adenovirus, is generated in insect cells in a baculovirus system and subsequently purified using sucrose density gradient and ion exchange column to eliminate low- and medium-molecular proteins and nucleic acids and to obtain an rDd fraction with purity of more than 90%; next, multiple copies of fusion protein including WW domains from fungal protein, particularly from yeast protein, extended by a protein- or peptide-based therapeutic substance comprising an antigen, preferably a human or animal vaccine, an anti-tumour, immunogenic or immunosuppressive peptide and optionally fusion protein including a WW domain from fungal protein, particularly from yeast protein, extended by a peptide/protein, lipid or polysaccharide determining specific tissue tropism, are attached by *in vitro* incubation or by simultaneous expression in the baculovirus system.

6. Method according to Claim 5, **characterized in that** the protein or peptide therapeutic substance comprises an M1 or HA influenza virus antigen, or fragments thereof.

7. Method according to Claim 5 or 6, **characterized in that** said fusion protein consists of influenza proteins M1, HA, or fragments thereof, genetically fused to a WW linker protein.

8. Method according to Claim 5, **characterized in that** the recombinant dodecahedron particle, rDd, is obtained in high yield in insect cells in a baculovirus system, wherein the yield is at least 8 mg rDd per 100 ml of cell culture.

9. A virus-like particle vector according to any of claims 1 to 4 for use in the delivery of therapeutic substances to human or animal tissues.

10. The virus-like particle vector for the use according to claim 9, wherein the protein or peptide therapeutic substance comprises an M1 or HA influenza virus antigen, or fragments thereof.

11. Pharmaceutical composition, **characterized in that** said composition contains a virus-like particle vector described in claims 1 to 4.

12. Composition according to Claim 11, **characterized in that** said composition is in the form of vaccine.

## Patentansprüche

1. Virus-ähnliches Vektorpartikel, **dadurch gekennzeichnet, dass** dieser Vektor ein rekombinantes Adenovirus-Dodecahedron-Partikel (Dd) ist, bestehend aus Pentonen oder Pentonbasis-Proteinen des Säuger-Adenovirus, insbesondere des humanen Adenovirus, wobei das Dd nicht-kovalent gebundene Fusionsproteine trägt, die viele WW-Domänen aus Pilz-Proteinen enthalten, insbesondere aus Hefe-Proteinen, günstigerweise aus dem Hefestamm *S. cerevisiae,* günstigerweise aus dem Rsp5-Protein, in genetischer Fusion mit einer antigenhaltigen therapeutischen Protein- oder Peptidsubstanz, günstigerweise Grippevirus-Antigen, und in dem diese WW-Domänen mit PPxZ-Motiven (Polin-Prolinbeliebige Aminosäure -Tyrosin) mit Dd reagieren.

2. Partikel nach Anspruch 1, **dadurch gekennzeichnet, dass** drei aus Rsp5-Protein stammende WW-Domänen im Tandem als Universalverbinder wirken, die das Anbinden vieler Kopien eines beliebigen Proteins oder Peptids an die Dodecahedrone ermöglichen.

3. Partikel nach Anspruch 1, in dem Fusionsprotein WW-Domänen an deren C-Schwanz enthält.

4. Partikel nach Anspruch 1, **dadurch gekennzeichnet, dass** ein Antigen-Protein ein Protein der Matrix M1 des Grippevirus oder Hämaglutinin HA oder deren Fragmente ist, in genetischer Fusion mit WW-Verbund-Protein.

5. Verfahren zur Herstellung eines virus-ähnlichen Vektorpartikels, das in den Ansprüchen 1 bis 4 beschrieben wird, **dadurch gekennzeichnet, dass** ein rekombinantes Adenovirus-Dodecahedron-Partikel, bestehend aus Pentonen oder Pentonbasis-Proteinen des Säuger-Adenovirus, insbesondere des humanen Adenovirus, in Insektenzellen im Bakulovirussystem hergestellt wird und anschließend unter Ausnutzung des Saccharosedichtegradienten und der Ionenaustauschsäule gereinigt wird, um Niedrig- und Mittelpartikelproteine und Nukleinsäuren zu eliminieren und eine rDd-Fraktion mit einem Reinheitsgrad über 90 % zu erhalten, und anschließend viele Kopien des Fusionsproteins, das die WW-Domäne aus Pilzprotein enthält, insbesondere Hefeprotein, verlängert durch die therapeutische Protein- oder Peptidsubstanz, die ein Antigen enthält, günstigerweise eine humane oder tierische Impfsubstanz, ein Antikrebsmittel, ein immunogenes oder immunsupressives Peptid und gegebenenfalls ein Fusionsprotein, das eine WW-Domäne aus Pilzprotein enthält, insbesondere aus Hefeprotein, verlängert durch Peptid/Protein, Lipid oder Polysaccharid, das den spezifischen Gewebetropismus bestimmt, durch *invitro-*Inkubation oder durch gleichzeitige Expression im Bakulovirussystem eingebunden werden.

6. Verfahren nach Anspruch 5, **dadurch gekennzeichnet, dass** eine therapeutische Protein- oder Peptidsubstanz ein Grippevirus-Antigen enthält: M1 oder HA oder dessen Fragmente.

7. Ein Verfahren nach Anspruch 5 oder 6, **dadurch gekennzeichnet, dass** dieses Fusionsprotein aus den Grippe-Proteinen besteht: M1, HA oder deren Fragmente, in genetischer Fusion mit WW-Verbund-Protein.

8. Verfahren nach Anspruch 5, **dadurch gekennzeichnet, dass** ein rekombinantes Dodecahedron-Partikel, rDd, mit hoher Kapazität in Insektenzellen im Bakulovirussystem erhalten wird, wobei die Kapazität mindestens 8 mg rDd pro 100 ml der Zellzucht beträgt.

9. Virus-ähnliches Vektorpartikel nach einem beliebigen der Ansprüche 1 bis 4, eingesetzt beim Einbringen von therapeutischen Substanzen in humane oder tierische Gewebe.

10. Virus-ähnliches Vektorpartikel zur Anwendung nach Anspruch 9, in dem eine therapeutische Protein- oder Peptidsubstanz ein Grippevirus-Antigen enthält: M1 oder HA, oder dessen Fragmente.

11. Pharmazeutische Komposition **dadurch gekennzeichnet, dass** diese Komposition das in den Ansprüchen 1 bis 4 beschriebene virus-ähnliche Vektorpartikel enthält.

12. Komposition nach Anspruch 11, **dadurch gekennzeichnet, dass** diese Komposition die Form eines Impfstoffes hat.

## Revendications

1. Le vecteur de particules (molécules) de type viral (virus-like particle (VLP)) **caractérisé en ce que** le vecteur est une molécule dodécaèdre d'adénovirus recombinant (Dd) composée de pentons ou de protéines de base de penton d'un adénovirus de mammifère, en particulier d'un adénovirus humain, qui porte des protéines non-covalentes de fusion fixées contenant de multiples domaines WW des protéines fongiques, en particulier des protéines de levure, de préférence d'une souche de levure *S. cerevisiae,* de préférence de la protéine Rsp5, génétiquement fusionnée à la substance thérapeutique à base de protéine ou peptide comprenant l'antigène, de préférence l'antigène du virus de la grippe et dans lequel ces domaines WW sont en interaction avec les motifs PPxZ (acide aminé polino-proline quelconque-tyrosine) avec Dd.

2. La molécule selon la revendication 1, **caractérisée en ce que** les trois domaines WW dérivés de la protéine Rsp5 agissent en tandem comme des adaptateurs universels permettant la fixation des copies multiples d'une protéine quelconque à un peptide ou à des dodécaèdres.

3. La molécule selon la revendication 1, dans laquelle la protéine de fusion contient les domaines WW à leur extrémité C.

4. La molécule selon la revendication 1, **caractérisée en ce que** la protéine d'antigène est une protéine de matrice M1 du virus de la grippe ou de l'hémagglutinine HA ou de leurs fragments en fusion génétique avec la protéine de liaison WW.

5. La méthode de production d'un vecteur de particules (molécules) de type viral, tel que décrite dans les revendications 1 à 4, **caractérisée en ce que** la molécule d'adénovirus dodécaèdre recombinante consistant de pentons ou de protéines de base de penton d'un mammifère, en particulier d'un humain, est produite dans des cellules d'insectes dans un système baculoviral (un système d'expression baculovirus), ensuite elle est purifiée en utilisant un gradient de densité de saccharose et de la colonne échangeuse d'ions pour éliminer les protéines à faible ou moyen poids moléculaire et des acides nucléiques, et pour obtenir une fraction de rDd de la pureté supérieure à 90 %, et ensuite de multiples copies de la protéine de fusion comprenant le domaine WW dérivé da la protéine fongique, en particulier, la protéine de levure, prolongée par une substance thérapeutique à base de protéine ou de peptide comprenant un antigène, de préférence un vaccin d'un humain ou d'un animal, un agent anticancéreux, un peptide immunogène ou immunosuppresseur et, éventuellement, une protéine de fusion comprenant un domaine WW de protéine fongique, en particulier de protéine de levure prolongée par un peptide / une protéine, un lipide ou un polysaccharide déterminant un tropisme tissulaire spécifique, sont fixés par l'incubation in vitro ou par co-expression dans le système baculovirus.

6. Le procédé selon la revendication 5, **caractérisé en ce que** l'agent thérapeutique protéique ou dérivé d'un peptide contient un antigène d'un virus de la grippe M1 ou HA ou ses fragments.

7. Le procédé selon la revendication 5 ou 6, **caractérisé en ce que** la protéine de fusion est constituée de protéines grippales M1, HA ou de leurs fragments, dans la fusion génétique avec la protéine de liaison WW.

8. Le procédé selon la revendication 5, **caractérisé en ce que** la molécule recombinante dodécaédrique rDd est obtenue avec un rendement élevé dans des cellules d'insectes dans le système baculoviral (un système d'expression baculovirus) - ce rendement est d'au moins 8 mg rDd par 100 ml de culture cellulaire.

9. Le vecteur de particules (molécules) de type viral selon l'une des revendications 1 à 4, pour utilisation dans l'administration d'agents thérapeutiques aux tissus humains ou animaux.

10. Le vecteur de particules (molécules) de type viral pour utilisation selon la revendication 9, dans lequel l'agent thérapeutique protéique ou dérivé d'un peptide contient un antigène, un virus de la grippe M1 ou HA ou leurs fragments.

11. La composition pharmaceutique, **caractérisée en ce qu'**elle comprend le vecteur de particules (molécules) de type viral décrit dans les revendications 1 à 4.

12. La composition selon la revendication 11, **caractérisée en ce qu'**elle se présente sous forme d'un vaccin.
